# EUROPEAN PATENT SPECIFICATION

(11) **EP 4 084 743 B1**
(45) Date of publication and mention of the grant of the patent: **12.03.2025**
(21) Application number: 20842582.7
(22) Date of filing: 24.12.2020
(51) Int. Cl.: A61F 2/46

(54) **SURGICAL INSTRUMENT BOLT**
BOLZEN FÜR CHIRURGISCHES INSTRUMENT
BOULON POUR INSTRUMENT CHIRURGICAL

(30) Priority: 30.12.2019 US 201916729822
(43) Date of publication of application: 09.11.2022
(73) Proprietor: DePuy Ireland Unlimited Company, County Cork (IE)
(72) Inventor: PEDDLE, Darron G., RAYNHAM, Massachusetts 02767-0350 (US)
(74) Representative: Murgitroyd & Company
(86) International application number: PCT/EP2020/087885
(87) International publication number: WO 2021/136748

(56) References cited:
- GB-A- 2 477 981
- US-A1- 2007 270 973
- US-A1- 2010 016 860
- US-A1- 2016 100 956

## Description

### TECHNICAL FIELD

The present disclosure relates generally to orthopaedic surgical instruments and, more particularly, to surgical instrument bolts for securing an orthopaedic surgical instrument to an orthopaedic prosthesis.

### BACKGROUND

Joint arthroplasty is a well-known surgical procedure by which a diseased and/or damaged natural joint is replaced by a prosthetic joint, which may include one or more orthopaedic prosthesis. For example, in a hip arthroplasty surgical procedure, a patient's natural hip ball and socket joint is partially or totally replaced by a prosthetic hip joint. Similarly, in a knee arthroplasty surgical procedure, a patient's natural knee joint is partially or totally replaced by a prosthetic knee joint.

One type of orthopaedic prostheses that may be used to replace a patient's joint are known as cementless orthopaedic prostheses. An orthopaedic surgeon implants cementless prostheses into a patient's boney anatomy by impacting the prosthesis into a corresponding bone of the patient using an orthopaedic prosthesis inserter. For example, a cementless acetabular prosthesis typically includes an acetabular cup outer shell, which is configured to be implanted into a patient's acetabulum. To do so, the orthopaedic surgeon may utilize an orthopaedic prosthesis inserter to impact the outer shell into the patient's acetabulum until the outer shell is sufficiently seated into the patient's surrounding bony anatomy. Additionally, other types of cementless orthopaedic prostheses may be implanted into a patient's bony anatomy in a similar fashion using associated orthopaedic surgical instruments.

Typically, the orthopaedic prosthesis is secured to the orthopaedic prosthesis inserter or other orthopaedic surgical instrument prior to impaction. To do so, one or more surgical instrument bolts may be used to temporarily attach the orthopaedic prosthesis to the orthopaedic prosthesis inserter. However, depending on the amount of bolt stretch exhibited by the surgical instrument bolt(s), the bolts may become loosened during impaction due to vibration, which may require the orthopaedic surgeon to repeatedly re-tighten the bolt(s). GB2477981 provides a surgical instrument according to the preamble of claim 1.

### SUMMARY

The invention concerns an orthopaedic surgical instrument for inserting an orthopaedic prosthesis into a bone of a patient as defined in claim 1.

In some embodiments, the inner surface further defines a first aperture on a top side of the bolt head opposite the bottom side and a second aperture on a bottom side of the threaded end of the bolt shaft. In such embodiments, the axial passageway extends from the first aperture to the second aperture such that the axial passageway extends completely through the surgical instrument bolt.

Alternatively, in other embodiments, the inner surface further defines an aperture on a top end of the bolt head, and the axial passageway is a blind passageway that extends from the aperture, through the bolt head, and into the shank of the bolt shaft. In such embodiments, the blind passageway may extend into the shank of the bolt shaft without extending into the threaded end of the bolt shaft.

In some embodiments, the inner surface further defines an aperture on a bottom side of the threaded end of the bolt shaft, and the axial passageway is a blind passageway that extends from the aperture into the bolt shaft. In such embodiments, the blind passageway extends from the aperture on the bottom side of the threaded end of the bolt shaft, through the threaded end, and into the shank of the bolt shaft without extending into the bolt head.

Additionally, in some embodiments, the axial passageway is embodied as an inner chamber located within the shank of the bolt shaft. In such embodiments, the inner chamber may not extend into the bolt head or into the threaded end of the bolt shaft.

According to background information, securing device for use with a surgical instrument includes a surgical instrument bolt having a bolt head and a bolt shaft extending away from a bottom side of the bolt head. The bolt shaft includes a threaded end having a plurality of bolt threads defined thereon, a shank located between the bolt head and the threaded end, and an inner surface defining an axial passageway that extends through at least a portion of the bolt shaft.

In the background information, the inner surface further defines a first aperture on a top side of the bolt head opposite the bottom side and a second aperture on a bottom side of the threaded end of the bolt shaft. In such embodiments, the axial passageway extends from the first aperture to the second aperture such that the axial passageway extends completely through the surgical instrument bolt.

Alternatively, in the background information, the inner surface further defines an aperture on a top end of the bolt head, and the axial passageway is a blind passageway that extends from the aperture, through the bolt head, and into the shank of the bolt shaft. In the background information, the blind passageway may extend into the shank of the bolt shaft without extending into the threaded end of the bolt shaft.

In some background information, the inner surface further defines an aperture on a bottom side of the threaded end of the bolt shaft, and the axial passageway is a blind passageway that extends from the aperture into the bolt shaft. In such background information, the blind passageway extends from the aperture on the bottom side of the threaded end of the bolt shaft, through the threaded end, and into the shank of the bolt shaft without extending into the bolt head.

Additionally, in some background information, the axial passageway is embodied as an inner chamber located within the shank of the bolt shaft. In such background information, the inner chamber may not extend into the bolt head or into the threaded end of the bolt shaft.

According to some embodiments , the orthopaedic surgical instrument for inserting an orthopaedic prosthesis into a bone of a patient includes a handle having an impaction plate and a shaft extending from the handle. The shaft includes a threaded end, a shank located between the handle and the threaded end, and an inner surface defining an axial passageway that extends through at least a portion of the shaft.

In some embodiments, the inner surface further defines an aperture on a bottom side of the threaded end and the axial passageway extends from the aperture into the shaft. In some embodiments, the axial passageway may be embodied as a blind passageway. Alternatively, in some embodiments, the axial passageway may be embodied as an inner chamber located within the shank of the shaft.

### BRIEF DESCRIPTION OF THE DRAWINGS

The concepts described herein are illustrated by way of example and not by way of limitation in the accompanying figures. For simplicity and clarity of illustration, elements illustrated in the figures are not necessarily drawn to scale. Where considered appropriate, reference labels have been repeated among the figures to indicate corresponding or analogous elements. The detailed description particularly refers to the accompanying figures in which:
FIG. 1 is a perspective view of an orthopaedic surgical instrument including an orthopaedic prosthesis inserter and a surgical instrument bolt configured to secure an orthopaedic prosthesis to the orthopaedic prosthesis inserter;
FIG. 2 is a perspective view of an embodiment of the surgical instrument bolt of the orthopaedic surgical instrument of FIG. 1;
FIG. 3 is a cross-sectional view of the surgical instrument bolt of FIG. 2;
FIG. 4 is a cross-sectional view of an alternative embodiment of the surgical instrument bolt of the orthopaedic surgical instrument of FIG. 1;
FIG. 5 is a cross-sectional view of another alternative embodiment of the surgical instrument bolt of the orthopaedic surgical instrument of FIG. 1;
FIG. 6 is a cross-sectional view of another alternative embodiment of the surgical instrument bolt of the orthopaedic surgical instrument of FIG. 1;
FIG. 7 is a side cross-sectional view of the orthopaedic prosthesis inserter and the surgical instrument bolt of the orthopaedic surgical instrument of FIG. 1 prior to securement of the orthopaedic prosthesis to the orthopaedic prosthesis inserter;
FIG. 8 is a side cross-sectional view of the orthopaedic prosthesis inserter and the surgical instrument bolt of the orthopaedic surgical instrument of FIG. 1 with the orthopaedic prosthesis secured to the orthopaedic prosthesis inserter via the surgical instrument bolt; and
FIG. 9 is a perspective view of another orthopaedic surgical instrument for inserting an orthopaedic prosthesis into a bone of a patient.

### DETAILED DESCRIPTION OF THE DRAWINGS

While the concepts of the present disclosure are susceptible to various modifications and alternative forms, specific embodiments thereof have been shown by way of example in the drawings and will be described herein in detail. It should be understood, however, that there is no intent to limit the concepts of the present disclosure to the particular forms disclosed, but on the contrary, the intention is to cover all modifications and alternatives consistent with the appended claims.

Terms representing anatomical references, such as anterior, posterior, medial, lateral, superior, inferior, etcetera, may be used throughout the specification in reference to the orthopaedic implants or prostheses and surgical instruments described herein as well as in reference to the patient's natural anatomy. Such terms have well-understood meanings in both the study of anatomy and the field of orthopaedics. Use of such anatomical reference terms in the written description and claims is intended to be consistent with their well-understood meanings unless noted otherwise.

References in the specification to "one embodiment," "an embodiment," "an illustrative embodiment," etc., indicate that the embodiment described may include a particular feature, structure, or characteristic, but every embodiment may or may not necessarily include that particular feature, structure, or characteristic. Moreover, such phrases are not necessarily referring to the same embodiment. Further, when a particular feature, structure, or characteristic is described in connection with an embodiment, it is submitted that it is within the knowledge of one skilled in the art to effect such feature, structure, or characteristic in connection with other embodiments whether or not explicitly described. Additionally, it should be appreciated that items included in a list in the form of "at least one A, B, and C" can mean (A); (B); (C); (A and B); (A and C); (B and C); or (A, B, and C). Similarly, items listed in the form of "at least one of A, B, or C" can mean (A); (B); (C); (A and B); (A and C); (B and C); or (A, B, and C).

In the drawings, some structural or method features may be shown in specific arrangements and/or orderings. However, it should be appreciated that such specific arrangements and/or orderings may not be required. Rather, in some embodiments, such features may be arranged in a different manner and/or order than shown in the illustrative figures. Additionally, the inclusion of a structural or method feature in a particular figure is not meant to imply that such feature is required in all embodiments and, in some embodiments, may not be included or may be combined with other features.

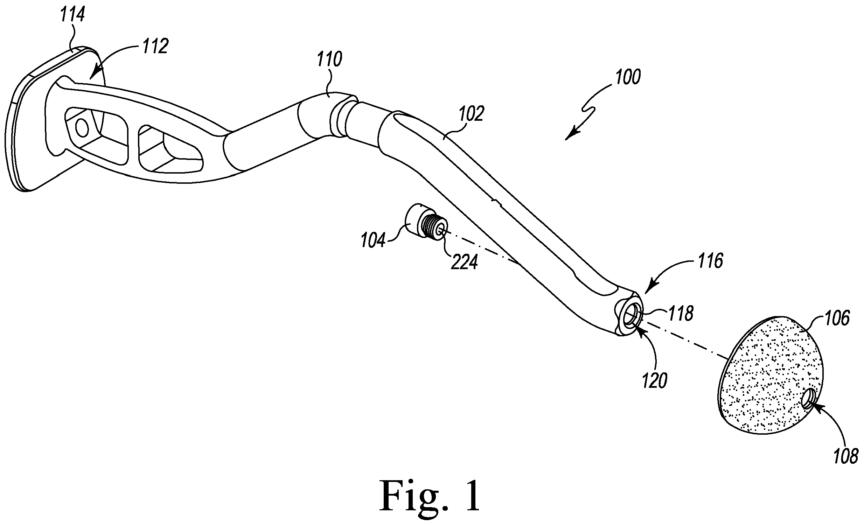

Referring now to FIG. 1, an orthopaedic surgical instrument 100 includes an orthopaedic prosthesis inserter 102 and a surgical instrument bolt 104. In use, as discussed in more detail below, the surgical instrument bolt 104 is usable to secure an orthopaedic prosthesis 106, such as an acetabular cup, to the orthopaedic prosthesis inserter 102 to facilitate implantation of the orthopaedic prosthesis 106 into a bone of a patient. To do so, the surgical instrument bolt 104 is received through a bolt passageway 120 of the orthopaedic prosthesis inserter 102 and screwed into a threaded aperture 108 of the orthopaedic prosthesis 106. Once so attached, an orthopaedic surgeon may align the orthopaedic prosthesis 106 to the patient's boney anatomy and impact the orthopaedic prosthesis 106 into a bone of the patient by striking an impaction plate 114 of the orthopaedic prosthesis inserter 102 with a surgical mallet or similar tool.

As discussed in more detail below, the surgical instrument bolt 104 includes an axial passageway 224 defined therethrough to increase the amount of bolt stretch exhibited by the surgical instrument bolt 104. Due to the increased bolt stretch, the tendency, frequency, or likelihood of the surgical instrument bolt 104 loosening during impaction is reduced. That is, the increased bolt stretch allows the surgical instrument bolt 104 to absorb vibrational forces caused during impaction without causing the threads of the surgical instrument bolt 104 to loosen. It should be appreciated that the inclusion of the axial passageway 224 increases the bolt stretch of the surgical instrument bolt 104 without the need to modify the diameter, length, or thread pitch of the surgical instrument bolt 104 relative to typical surgical instrument bolts used with the orthopaedic prosthesis inserter 102 and orthopaedic prosthesis 106. As such, in the illustrative embodiment, the surgical instrument bolt 104 may be used with typical orthopaedic prosthesis inserters and orthopaedic prostheses without modification thereof. Of course, in other embodiments, the surgical instrument bolt 104 may also include a modified diameter, length, and/or thread pitch relative to typical orthopaedic surgical bolts used with the orthopaedic prosthesis inserter 102.

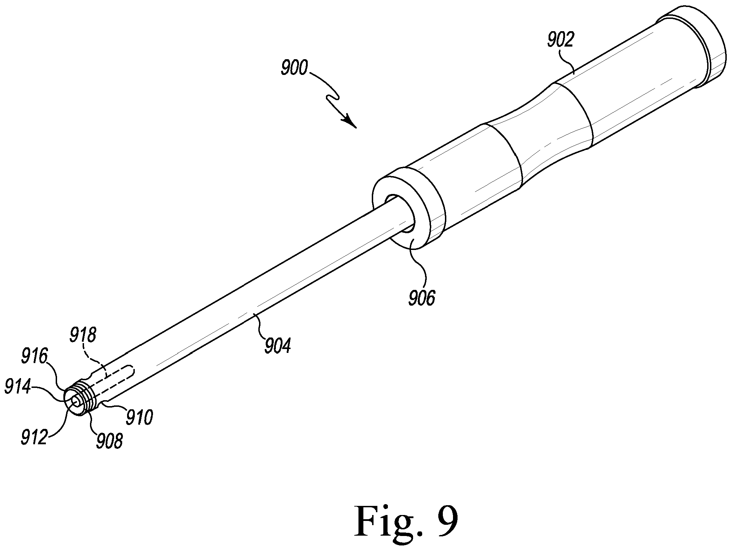

The orthopaedic prosthesis inserter 102 may be embodied as any type of orthopaedic prosthesis inserter in which a surgical instrument bolt is used to secure an orthopaedic prosthesis to the inserter. In the illustrative embodiment, as shown in FIG. 1, the orthopaedic prosthesis inserter 102 includes a curved body 110 having a distal end 112 to which the impaction plate 114 attached, integrally or separately. The impaction plate 114 is shaped and sized to provide a striking surface to receive impacts from an orthopaedic surgical mallet or similar tool during impaction of the orthopaedic prosthesis 106. Although the body 110 of the orthopaedic prosthesis inserter 102 is illustratively curved to allow positioning of the inserter 102 around the patient's anatomy, the orthopaedic prosthesis inserter 102 may have a body that is substantially straight or otherwise non-curved, similar to the orthopaedic surgical inserter 900 described below in regard to FIG. 9.

The body 110 of the orthopaedic prosthesis inserter 102 also includes a proximal end 116, opposite the distal end 112, which includes an inner surface 118 that defines the bolt passageway 120. The bolt passageway 120 is sized to receive the surgical instrument bolt 104. According to the invention, the inner surface 118 is threaded, and the surgical instrument bolt 104 may be threaded into the inner surface 118. The threads of the inner surface 118 are sized such that, when threaded through the inner surface 118, a threaded end of the surgical instrument bolt 104 extends out of the bolt passageway 120 from the distal end 112 and the surgical instrument bolt 104 is free to rotate within the bolt passageway 120 while being retained therein. In other examples that do not form part of the present invention, however, the bolt passageway 120 may not be threaded.

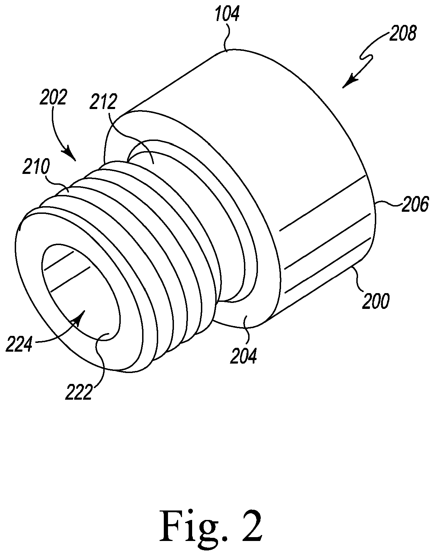

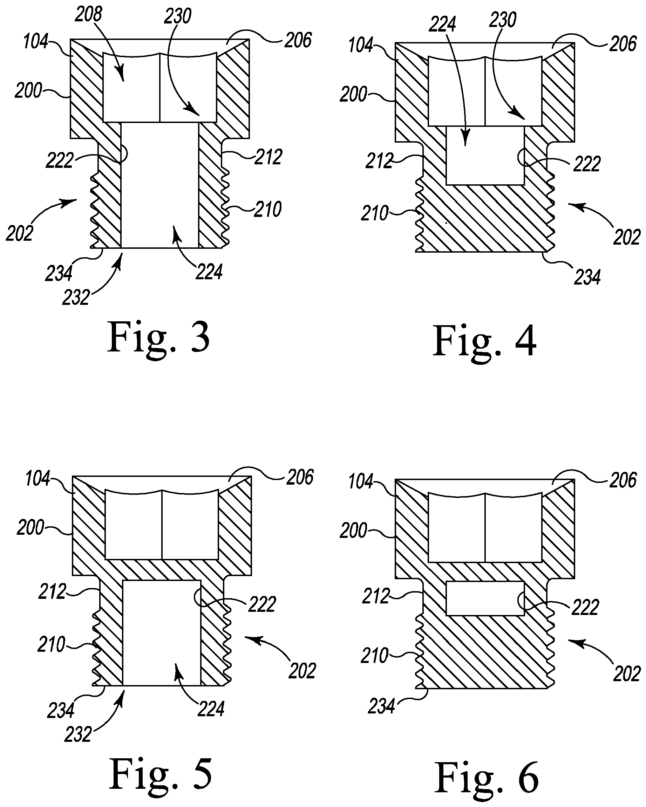

Referring now to FIGS. 2 and 3, the illustrative surgical instrument bolt 104 includes a bolt head 200 and a bolt shaft 202 that extends away from a bottom side 204 of the bolt head 200. The bolt head 200 also includes a top side 206 on which a tool aperture 208 is defined. The tool aperture 208 is shaped to receive a corresponding bolt tool (e.g, an Allen wrench) to allow the surgical instrument bolt 104 to be threaded into the orthopaedic prosthesis 106 as discussed in more detail below. Of course in other embodiments, the surgical instrument bolt 104 may not include the tool aperture 208 and, in such embodiments, the bolt head 200 may be shaped (e.g., hexagonal) to facilitate the threading of the surgical instrument bolt 104 into the orthopaedic prosthesis 106 using a corresponding tool, such as a bolt driver.

The bolt shaft 202 includes a threaded end 210 and a shank 212 located between the bolt head 200 and the threaded end 210. In the illustrative embodiment of FIG. 3, the shank 212 is not threaded, unlike the threaded end 210. However, in other examples, the shank 212 may be threaded (e.g., the entire bolt shaft 202 may be threaded) and, in such examples, the shank 212 corresponds to that section of the bolt shaft 202 that does not engage with the mating threads of the threaded aperture 108 of the orthopaedic prosthesis 106.

The bolt shaft 202 also includes an inner surface 222 that defines the axial passageway 224, which extends through at least the shank 212 of the bolt shaft 202. It should be appreciated that because the axial passageway 224 extends through the shank 212 of the bolt shaft 202, the cross-sectional area of the shank 212 is reduced, which increases the bolt stretch of the surgical instrument bolt 104.

In the illustrative embodiment of FIGS. 2 and 3, the axial passageway 224 extends completely through the bolt shaft 202. That is, the inner surface 222 further defines a top aperture 230 on the top side 206 of the bolt head 200, which is illustratively inside the tool aperture 208, and a bottom aperture 232 on a bottom side 234 of the threaded end 210. The axial passageway 224 extends from the top aperture 230, through the shank 212 and the threaded end 210, and to the bottom aperture 232.

In other embodiments, the axial passageway 224 may be embodied as a blind passageway that does not extend completely through the bolt shaft 202. For example, as shown in FIG. 4, the axial passageway 224 extends from the top aperture 230 on the top side 206 of the bolt head 200, through the shank 212, and terminates near the threaded end 210 without extending therethrough. As such, in the embodiment of FIG. 4, the axial passageway 224 includes the top aperture 230 but does not include the bottom aperture 232 on the bottom side 234 of the threaded end 210.

Alternatively, as shown in FIG. 5, the axial passageway 224 may be embodied as a blind passageway that extends from the bottom aperture 232 on the bottom side 234 of the threaded end 210, through the threaded end 210 and the shank 212, and terminates just below the bolt head 200 without extending therethrough. As such, in the embodiment of FIG. 5, the axial passageway 224 includes the bottom aperture 232 but does not include the top aperture 230 on the top side 206 of the bolt head 200.

In other embodiments, as show in FIG. 6, the axial passageway 224 may be embodied as an inner chamber located in the shank 212 of the bolt shaft 202. In such embodiments, the axial passageway 224 does not include the top aperture 230 or the bottom aperture 232. Rather, the axial passageway 224 forms a sealed void within the shank 212.

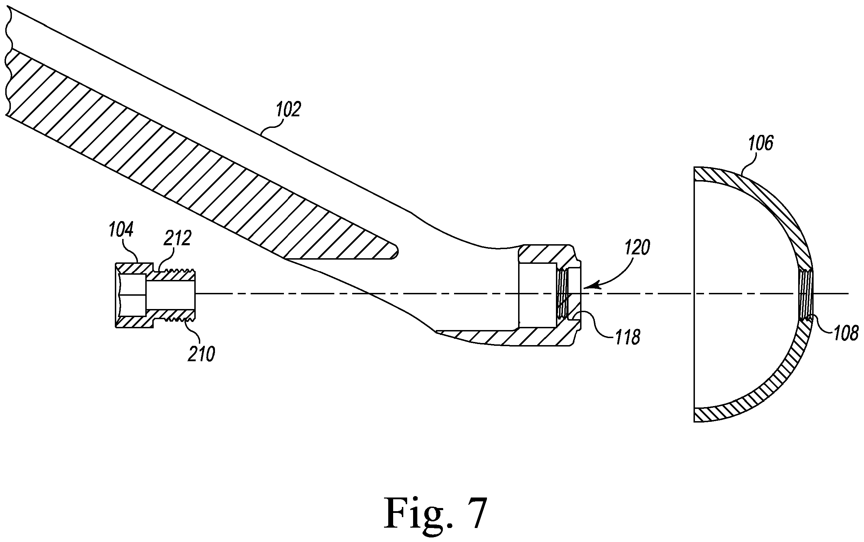

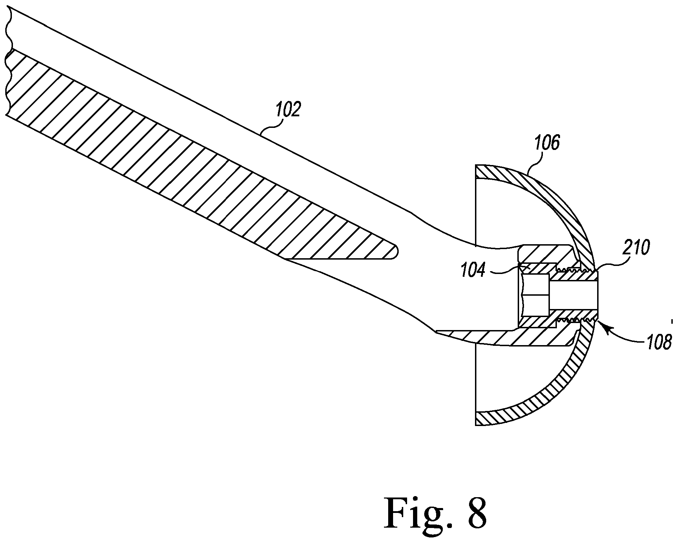

Referring now to FIGS. 7 and 8, in use, an orthopaedic surgeon may secure the orthopaedic prosthesis 106 to the orthopaedic prosthesis inserter 102 by inserting the surgical instrument bolt 104 into the bolt passageway 120 of the orthopaedic prosthesis inserter 102 as indicated in FIG 7. The surgical instrument bolt 104 is positioned in the bolt passageway 120 such that the threaded end 210 of the surgical instrument bolt 104 extends out of the bolt passageway 120. The surgical instrument bolt 104 is threaded into the bolt passageway 120 until the shank 212 extends though the threaded inner surface 118 and the threaded end 210 of the surgical instrument bolt 104 extends from the bolt passageway 120. The orthopaedic surgeon may then thread the surgical instrument bolt 104 into the threaded aperture 108 of the orthopaedic prosthesis inserter 102 as shown in FIG. 8 to secure the orthopaedic prosthesis inserter 102 onto the orthopaedic prosthesis inserter 102. Once so secured, the orthopaedic surgeon may position the orthopaedic prosthesis 106 to the patient's boney anatomy and impact the orthopaedic prosthesis 106 into a bone of the patient by repeatedly striking the impaction plate 114 of the orthopaedic prosthesis inserter 102 until the orthopaedic prosthesis 106 is properly seated.

Although the orthopaedic prosthesis 106 is illustratively shown as an acetabular cup prosthesis, it should be appreciated that the surgical instrument bolt 104 may be used with other types of prostheses to secure those prostheses to the orthopaedic prosthesis inserter 102 or other prosthesis inserter. Additionally, the surgical instrument bolt 104 may be used with surgical tools, such as an orthopaedic broach, to secure such surgical tools to an associated orthopaedic inserter, impactor, or other surgical instrument. Furthermore, it should be appreciated that the surgical instrument bolt 104 may be used with other types of orthopaedic surgical instruments, other than the orthopaedic prosthesis inserter 102. That is, the surgical instrument bolt 104 may be useful with any orthopaedic surgical instrument to replace a typical bolt that may loosen due to vibrational forces to improve the bolt stretch typically exhibited by such bolts and thereby reduce the likelihood of loosening.

Referring now to FIG. 9, it should be appreciated that the inclusion of the axial passageway 224 described above in regard to the surgical instrument bolt 104 may be applicable to other orthopaedic surgical instruments that are exposed to impacts or vibrational forces during use to improve the securement of such orthopaedic surgical instruments. For example, as shown in FIG. 9, an orthopaedic prosthesis inserter 900 includes a handle 902 and a shaft 904 extending from a bottom side 906 of the handle 902. The shaft 904 includes a threaded end 908 and a shank 910 located between the threaded end 908 and the handle 902. The threaded end 908 is configured to be screwed directly into a corresponding orthopaedic prosthesis, such as the orthopaedic prosthesis 106, without the use of a surgical instrument bolt. As such, the orthopaedic prosthesis inserter 900 may be attached to an orthopaedic prosthesis by threading the threaded end 908 into a corresponding threaded aperture of the orthopaedic prosthesis. Once so attached, an orthopaedic surgeon may align the orthopaedic prosthesis to the patient's boney anatomy and impact the orthopaedic prosthesis into a bone of the patient by striking the handle 902.

To reduce the likelihood of loosening of the orthopaedic prosthesis inserter 900 and the orthopaedic prosthesis during impaction, the shaft 904 of the orthopaedic prosthesis inserter 900 includes an inner surface 912 that defines an aperture 914 on an end 916 of the threaded end 908 and an axial passageway 918 that extends into the shaft 904 from the aperture 914. Similar to the surgical instrument bolt 104 described above, the axial passageway 918 extends through the shank 910 of the shaft 904. As such, the cross-sectional area of the shank 910 is reduce, which increases the bolt stretch of the threaded end 908 and reduces the likelihood that the threaded end 908 loosens during impaction.

Although the orthopaedic surgical instrument of FIG. 9 is illustrated as an orthopaedic prosthesis inserter, it should be appreciated that the orthopaedic surgical instrument of FIG. 9 may be embodied as other types of orthopaedic surgical instruments in other embodiments. That is, the illustrative axial passageway 918 may be incorporated in any other orthopaedic surgical instrument having a threaded end to improve the bolt stretch of that threaded end and reduce the likelihood of the orthopaedic surgical instrument loosening during use.

While certain illustrative embodiments have been described in detail in the drawings and the foregoing description, such an illustration and description is to be considered as exemplary and not restrictive in character, it being understood that only illustrative embodiments have been shown and described and that all changes and modifications that come within the scope of the present invention are desired to be protected.

There are a plurality of advantages of the present disclosure arising from the various features of the method, apparatus, and system described herein. It will be noted that alternative embodiments of the apparatus of the present invention may not include all of the features described yet still benefit from at least some of the advantages of such features. Those of ordinary skill in the art may readily devise their own implementations of the apparatus that incorporate one or more of the features of the present invention and fall within the scope of the present invention as defined by the appended claims.

## Claims

1. An orthopaedic surgical instrument [100] for inserting an orthopaedic prosthesis [106] into a bone of a patient, the orthopaedic surgical instrument [100] comprising:
an orthopaedic prosthesis inserter [102] having a body and a bolt passageway [120] defined through the body; and
a surgical instrument bolt [104] configured to be received in the bolt passageway [120] of the body of the orthopaedic prosthesis inserter, wherein the surgical instrument bolt [104] comprises a bolt head [200] and a bolt shaft [202] extending away from a bottom side [204] of the bolt head [200], wherein the bolt shaft [202] comprises a (i) a threaded end [[210] configured to extend out of a distal end of the orthopaedic prosthesis inserter, (ii) a shank [212] located between the bolt head [200] and the threaded end [210], and (iii) an inner surface [222] defining an axial passageway [224] that extends through at least a portion of the bolt shaft [202];
**characterized in that**:
an inner surface [118] of the bolt passageway [120] is threaded;
wherein the surgical instrument bolt [104] is configured to be threaded into the inner surface [118] of the bolt passageway, the threads of the inner surface [118] being sized such that, when threaded through the inner surface [118], the surgical instrument bolt [104] is free to rotate within the bolt passageway [120] while being retained therein.

2. The orthopaedic surgical instrument [100] of claim 1, wherein the inner surface [118] further defines a first aperture [208] on a top side [206] of the bolt head [200] opposite the bottom side [204] and a second aperture [232] on a bottom side [204] of the threaded end [210] of the bolt shaft [202], wherein the axial passageway [224] extends from the first aperture [208] to the second aperture [232] such that the axial passageway [224] extends completely through the surgical instrument bolt [104].

3. The orthopaedic surgical instrument [100] of claim 1, wherein the inner surface [118] further defines an aperture on a top end of the bolt head [200], wherein the axial passageway [224] comprises a blind passageway that extends from the aperture, through the bolt head [200], and into the shank [212] of the bolt shaft [202].

4. The orthopaedic surgical instrument [100] of claim 3, wherein the blind passageway extends into the shank [212] of the bolt shaft [202] without extending into the threaded end [210] of the bolt shaft [202].

5. The orthopaedic surgical instrument [100] of claim 1, wherein the inner surface [118] further defines an aperture on a bottom side [204] of the threaded end [210] of the bolt shaft [202] and the axial passageway [224] comprises a blind passageway that extends from the aperture into the bolt shaft [202].

6. The orthopaedic surgical instrument [100] of claim 5, wherein the blind passageway extends from the aperture on the bottom side [204] of the threaded end [210] of the bolt shaft [202], through the threaded end [210], and into the shank [212] of the bolt shaft [202] without extending into the bolt head.

7. The orthopaedic surgical instrument [100] of claim 1, wherein the axial passageway [224] comprises an inner chamber located within the shank [212] of the bolt shaft [202].

8. The orthopaedic surgical instrument [100] of claim 7, wherein the inner chamber does not extend into the bolt head [200] or into the threaded end [210] of the bolt shaft [202].

9. The orthopaedic surgical instrument [100] of any preceding claim, comprising:
a handle [902] having an impaction plate [114]; and
a shaft [904] extending from the handle [902], wherein the shaft [904] includes a threaded end [908], a shank [910] located between the handle 902] and the threaded end [908], and an inner surface [912] defining an axial passageway [918] that extends through at least a portion of the shaft [904].

10. The orthopaedic surgical instrument [100] of claim 9, wherein the inner surface [912] further defines an aperture [914] on a bottom side of the threaded end [908] and the axial passageway [918] extends from the aperture [914] into the shaft [904].

11. The orthopaedic surgical instrument [100] of claim 10, wherein the axial passageway [918] comprises a blind passageway.

12. The orthopaedic surgical instrument [100] of claim 9, wherein the axial passageway [914] comprises an inner chamber located within the shank [910] of the shaft [904].

## Patentansprüche

1. Orthopädisches chirurgisches Instrument [100] zum Einsetzen einer orthopädischen Prothese [106] in einen Knochen eines Patienten, wobei das orthopädische chirurgische Instrument [100] umfasst:
einen orthopädischen Protheseneinsetzer [102] mit einem Körper und einem Bolzendurchgang [120], der durch den Körper hindurch definiert ist; und
einen chirurgischen Instrumentenbolzen [104], der konfiguriert ist, in dem Bolzendurchgang [120] des Körpers des orthopädischen Protheseneinsetzers aufgenommen zu sein bzw. zu werden, wobei der chirurgische Instrumentenbolzen [104] einen Bolzenkopf [200] und einen Bolzenschaft bzw. eine Bolzenwelle [202] umfasst, der bzw. die sich von einer unteren Seite [204] des Bolzenkopfes [200] weg erstreckt, wobei die Bolzenwelle [202] (i) ein Gewindeende [210], das konfiguriert ist, sich aus einem distalen Ende des orthopädischen Protheseneinsetzers zu erstrecken, (ii) einen Schaft [212], der sich zwischen dem Bolzenkopf [200] und dem Gewindeende [210] befindet, und (iii) eine Innenfläche bzw. -oberfläche [222] umfasst, die einen axialen Durchgang [224] definiert, der sich durch zumindest einen Abschnitt der Bolzenwelle [202] erstreckt;
**dadurch gekennzeichnet, dass**:
eine Innenfläche bzw. -oberfläche [118] des Bolzendurchgangs [120] mit Gewinde versehen ist;
wobei der chirurgische Instrumentenbolzen [104] konfiguriert ist, in die Innenfläche [118] des Bolzendurchgangs eingeschraubt zu werden, wobei die Gewinde der Innenfläche [118] so bemessen sind, dass der chirurgische Instrumentenbolzen [104], wenn er durch die Innenfläche [118] geschraubt ist bzw. wird, sich frei innerhalb des Bolzendurchgangs [120] drehen kann, während er darin zurückgehalten wird.

2. Orthopädisches chirurgisches Instrument [100] nach Anspruch 1, wobei die Innenfläche [118] ferner eine erste Öffnung [208] an einer oberen Seite [206] des Bolzenkopfes [200] gegenüberliegend bzw. entgegengesetzt zu der unteren Seite [204] und eine zweite Öffnung [232] an einer unteren Seite [204] des Gewindeendes [210] der Bolzenwelle [202] definiert, wobei sich der axiale Durchgang [224] von der ersten Öffnung [208] zu der zweiten Öffnung [232] erstreckt, so dass sich der axiale Durchgang [224] vollständig durch den chirurgischen Instrumentenbolzen [104] erstreckt.

3. Orthopädisches chirurgisches Instrument [100] nach Anspruch 1, wobei die Innenfläche [118] ferner eine Öffnung an einem oberen Ende des Bolzenkopfes [200] definiert, wobei der axiale Durchgang [224] einen Blind- bzw. Sackdurchgang umfasst, der sich von der Öffnung durch den Bolzenkopf [200] und in den Schaft [212] der Bolzenwelle [202] erstreckt.

4. Orthopädisches chirurgisches Instrument [100] nach Anspruch 3, wobei sich der Sackdurchgang in den Schaft [212] der Bolzenwelle [202] erstreckt, ohne sich in das Gewindeende [210] der Bolzenwelle [202] zu erstrecken.

5. Orthopädisches chirurgisches Instrument [100] nach Anspruch 1, wobei die Innenfläche [118] ferner eine Öffnung an einer unteren Seite [204] des Gewindeendes [210] der Bolzenwelle [202] definiert und der axiale Durchgang [224] einen Sackdurchgang umfasst, der sich von der Öffnung in die Bolzenwelle [202] erstreckt.

6. Orthopädisches chirurgisches Instrument [100] nach Anspruch 5, wobei sich der Sackdurchgang von der Öffnung an der unteren Seite [204] des Gewindeendes [210] der Bolzenwelle [202] durch das Gewindeende [210] und in den Schaft [212] der Bolzenwelle [202] erstreckt, ohne sich in den Bolzenkopf zu erstrecken.

7. Orthopädisches chirurgisches Instrument [100] nach Anspruch 1, wobei der axiale Durchgang [224] eine Innenkammer umfasst, die sich innerhalb des Schafts [212] der Bolzenwelle [202] befindet.

8. Orthopädisches chirurgisches Instrument [100] nach Anspruch 7, wobei sich die Innenkammer nicht in den Bolzenkopf [200] oder in das Gewindeende [210] der Bolzenwelle [202] erstreckt.

9. Orthopädisches chirurgisches Instrument [100] nach einem der vorhergehenden Ansprüche, umfassend:
einen Griff [902] mit einer Impaktions- bzw. Einschlagplatte [114]; und
einen Schaft bzw. eine Welle [904], der bzw. die sich von dem Griff [902] erstreckt, wobei die Welle [904] ein Gewindeende [908], einen Schaft [910], der sich zwischen dem Griff [902] und dem Gewindeende [908] befindet, und eine Innenfläche [912] enthält, die einen axialen Durchgang [918] definiert, der sich durch zumindest einen Abschnitt der Welle [904] erstreckt.

10. Orthopädisches chirurgisches Instrument [100] nach Anspruch 9, wobei die Innenfläche [912] ferner eine Öffnung [914] an einer unteren Seite des Gewindeendes [908] definiert und sich der axiale Durchgang [918] von der Öffnung [914] in die Welle [904] erstreckt.

11. Orthopädisches chirurgisches Instrument [100] nach Anspruch 10, wobei der axiale Durchgang [918] einen Blind- bzw. Sackdurchgang umfasst.

12. Orthopädisches chirurgisches Instrument [100] nach Anspruch 9, wobei der axiale Durchgang [914] eine Innenkammer umfasst, die sich innerhalb des Schafts [910] der Welle [904] befindet.

## Revendications

1. Instrument chirurgical orthopédique (100) pour l'insertion d'une prothèse orthopédique (106) dans un os d'un patient, l'instrument chirurgical orthopédique (100) comprenant :
un porte-prothèse orthopédique (102) présentant un corps et un passage de boulon (120) défini à travers le corps ; et
un boulon d'instrument chirurgical (104) configuré pour être reçu dans le passage de boulon (120) du corps du porte-prothèse orthopédique, dans lequel le boulon d'instrument chirurgical (104) comprend une tête de boulon (200) et une tige de boulon (202) s'étendant à l'écart d'un côté inférieur (204) de la tête de boulon (200), dans lequel la tige de boulon (202) comprend (i) une extrémité filetée (210) configurée pour s'étendre hors d'une extrémité distale du porte-prothèse orthopédique,
(ii) un fût (212) situé entre la tête de boulon (200)
et l'extrémité filetée (210), et (iii) une surface interne (222) définissant un passage axial (224) qui s'étend à travers au moins une partie de la tige de boulon (202) ;
**caractérisé en ce que** :
une surface interne (118) du passage de boulon (120) est filetée ;
dans lequel
le boulon d'instrument chirurgical (104) est configuré pour être vissé dans la surface interne (118) du
passage de boulon, les filetages de la surface interne (118) étant dimensionnés de sorte que, lorsqu'ils sont introduits à travers la surface interne (118), le boulon d'instrument chirurgical (104) puisse tourner librement à l'intérieur du passage de boulon (120) tout en étant retenu à l'intérieur de celui-ci.

2. Instrument chirurgical orthopédique (100) selon la revendication 1, dans lequel la surface interne (118) définit en outre une première ouverture (208) sur un côté supérieur (206) de la tête de boulon (200) opposé au côté inférieur (204) et une seconde ouverture (232) sur un côté inférieur (204) de l'extrémité filetée (210) de la tige de boulon (202), dans lequel le passage axial (224) s'étend de la première ouverture (208) à la seconde ouverture (232) de sorte que le passage axial (224) s'étende entièrement à travers le boulon d'instrument chirurgical (104).

3. Instrument chirurgical orthopédique (100) selon la revendication 1, dans lequel la surface interne (118) définit en outre une ouverture sur une extrémité supérieure de la tête de boulon (200), dans lequel le passage axial (224) comprend un passage aveugle qui s'étend depuis l'ouverture, à travers la tête de boulon (200), et dans le fût (212) de la tige de boulon (202).

4. Instrument chirurgical orthopédique (100) selon la revendication 3, dans lequel le passage aveugle s'étend dans le fût (212) de la tige de boulon (202) sans s'étendre dans l'extrémité filetée (210) de la tige de boulon (202).

5. Instrument chirurgical orthopédique (100) selon la revendication 1, dans lequel la surface interne (118) définit en outre une ouverture sur un côté inférieur (204) de l'extrémité filetée (210) de la tige de boulon (202) et le passage axial (224) comprend un passage aveugle qui s'étend depuis l'ouverture dans la tige de boulon (202).

6. Instrument chirurgical orthopédique (100) selon la revendication 5, dans lequel le passage aveugle s'étend depuis l'ouverture sur le côté inférieur (204) de l'extrémité filetée (210) de la tige de boulon (202), à travers l'extrémité filetée (210), et dans le fût (212) de la tige de boulon (202) sans s'étendre dans la tête de boulon.

7. Instrument chirurgical orthopédique (100) selon la revendication 1, dans lequel le passage axial (224) comprend une chambre interne située à l'intérieur du fût (212) de la tige de boulon (202).

8. Instrument chirurgical orthopédique (100) selon la revendication 7, dans lequel la chambre interne ne s'étend ni dans la tête de boulon (200), ni dans l'extrémité filetée (210) de la tige de boulon (202).

9. Instrument chirurgical orthopédique (100) selon une quelconque revendication précédente, comprenant :
un manche (902) présentant une plaque d'impaction (114) ; et
une tige (904) s'étendant à partir du manche (902), dans lequel la tige (904) inclut une extrémité filetée (908), un fût (910) situé entre le manche (902) et l'extrémité filetée (908), et une surface interne (912) définissant un passage axial (918) qui s'étend à travers au moins une partie de la tige (904).

10. Instrument chirurgical orthopédique (100) selon la revendication 9, dans lequel la surface interne (912) définit en outre une ouverture (914) sur un côté inférieur de l'extrémité filetée (908) et le passage axial (918) s'étend depuis l'ouverture (914) dans la tige (904).

11. Instrument chirurgical orthopédique (100) selon la revendication 10, dans lequel le passage axial (918) comprend un passage aveugle.

12. Instrument chirurgical orthopédique (100) selon la revendication 9, dans lequel le passage axial (914) comprend une chambre interne située à l'intérieur du fût (910) de la tige (904).
